# EUROPEAN PATENT SPECIFICATION

(11) **EP 1 140 035 B1**
(45) Date of publication and mention of the grant of the patent: **28.05.2003**
(21) Application number: 99963595.6
(22) Date of filing: 17.12.1999
(51) Int. Cl.: A61K 9/46, A61K 31/295

(54) **METHOD FOR PREPARING AN EFFERVESCENT GRANULATE COMPRISING IRON GLUCONATE, GRANULATE THUS OBTAINED AND TABLETS CONTAINING THE SAME**
VERFAHREN ZUR HERSTELLUNG EINES BRAUSEGRANULATES MIT EISENGLUCONAT, DAMIT HERGESTELLTE GRANULATE UND DIESE ENTHALTENDE TABLETTEN
PROCEDE DE PREPARATION D'UN GRANULE EFFERVESCENT COMPRENANT DU GLUCONATE DE FER, GRANULE AINSI OBTENU ET COMPRIMES LE COMPRENANT

(30) Priority: 24.12.1998 IT MI982821
(43) Date of publication of application: 10.10.2001
(73) Proprietor: E-PHARMA TRENTO S.p.A, 38040 Trento Ravina (TN) (IT)
(72) Inventor: CATALANO, Riccardo, I-38030 Castello, Molina di Fiemme (IT)
(74) Representative: Marchi, Massimo, Dr.
(86) International application number: EP9910210
(87) International publication number: WO00038657

(56) References cited:
- EP-A- 0 673 644
- DE-A- 3 632 334
- FR-A- 2 537 872
- GB-A- 1 338 071

## Description

The present invention relates to a method for preparing an effervescent granulate comprising ferrous gluconate, to the granulate thus obtained and to tablets containing the same.

Effervescent pharmaceutical forms containing ferrous gluconate are known.

So far, the suggested methods for preparing them such as, for example, the method described in patent EP-0 673 644-A, do not solve all the problems associated with the manufacture of effervescent granulates containing divalent iron, since it has been found that, in a number of cases, the granulate does not form and/or the divalent iron has a tendency to become oxidized to trivalent iron.

Apparently, these drawbacks are not encountered in processes based on so-called "passivation", such as the process described in European patent EP-B-0 076 340, which involves the thermal treatment at 30-100°C of a mixture in powder or granule form formed from an acid and from an acid carbonate and/or from a carbonate, as effervescent components, in a closed system under vacuum. According to this process, after optionally pretreating the acid at high temperature, it is mixed with the required amount of acid carbonate and/or carbonate and this mixture is then subjected to a treatment under vacuum and at a temperature of from 30 to 100°C, and preferably from 40 to 80°C, at which temperature the mixture is mixed with a polar solvent such as water, methanol, ethanol or mixtures thereof. After this step, the aggregates thus obtained are fragmented into particles of the desired size, the desired additives can be added and the resulting mixture can optionally be converted into tablets. This process is characterized in that, in order to passivate the surface of at least one of the components of the reaction and thus to reduce the components to a state of high inertia with respect to the reaction, a precise amount of polar solvent, of up to 7% of the mass considered as mass to be treated, is added to the mixture during the treatment under vacuum. The pressure difference caused by the evolvement of carbon dioxide by means of the addition of solvent during the reaction is set at up to a maximum of 1000 mbar, the volume and mass of carbon dioxide released are determined from this difference in pressure, and the thermal treatment is repeated, after quick drying of the mixture, as many times as necessary to obtain a passivation of the surface as indicated by an appreciable deceleration of the reaction and of the evolvement of gas.

However, it is easy to observe that this process is particularly complicated when it is desired to strictly monitor all of the numerous parameters involved in the reaction and that, when the polar solvent is water, the amount thereof is quite considerable. Moreover, if water is not used, the problem arises of recovering the organic polar solvent used.

GB-A-1 338 071 relates to a pharmaceutical composition comprising a non-toxic iron salt and an effervescent couple, wherein all or part of the acidic component of the effervescent couple has its surface properties modified by providing thereon a pharmacologically acceptable coating material which is adapted to make the coated acid particles substantially stable in the presence of an alkali component in the dry state and which is water soluble. Said water soluble coating material is an alkali or an alkaline hearth metal salt of the acidic component.

FR-A-2 537 872 relates to a wet granulation process by the fluidisedl bed technique. The three operations: premixing of the powders, triggering of the effervescent reaction and stopping this reaction, are performed in the same single apparatus called a granulator-drier.

DE-A-3 632 334 relates to an iron product which can be administered orally and contains at least one ferro salt which is soluble in water and whose anion is physiologically tolerated, such as iron gluconate, contains ascorbic acid and contains at least one CO₂-providing compound such as sodium bicarbonate, sodium carbonate or the like, as well as, where appropriate, sweetener(s), flavouring(s) and/or colorant(s). The product also contains at least one further weak physiologically tolerated acid with a lower pK1 than ascorbic acid.

The object of the present invention is to develop a process for preparing an effervescent granulate based on ferrous gluconate in which oxidation of the divalent iron and "passivation" of the granulate are substantially prevented. It is thus necessary for this process to Involve the smallest possible amount of water and to have few critical parameters, which can easily be kept under control.

According to a first aspect of the present invention, this object is achieved by means of a method according to claim 1.

Throughout the present description and the claims
- the expression "substantially constant" indicates that the temperature is maintained within a narrow range (about ± 3°C); the reason for this is that it has been found that above 38°C the granulate has a tendency not to form, while below 32°C the water has a tendency to be deposited on the surface of the granulate, thus triggering effervescence;
- the expression "desired features" of the granulate indicates a residual moisture content of ≤ 0.5%; an average particle size of between 100 and 500 µ; a run-out time of the granulate (100 g) from a glass funnel (walls inclined at 60° and outlet diameter of 9 mm) of < 20 sec., preferably < 18 sec., a tangent of the angle of rest of ≤ 1; a Carr index of ≤ 20; and an apparent specific weight of between 0.7 and 1.0 g/ml.

Preferably, the magnitude of the air flow rate in step I is between 3200 and 3800 m³/hour for each batch of about 265 kg of mixture to be granulated.

The flow rate of nebulized water in step II is preferably between 500 and 2000 ml/min, and even more preferably it is about 1000 ml/min. The pressure of the said flow is, in turn, preferably about 3 atmospheres.

Advantageously, the amount of water used for the granulation does not exceed about 2% by weight of the mass to be granulated. This is because using a small amount of water makes it possible to minimize not only any oxidation of the divalent iron, but also the trigger of the effervescence on the surface of the granulate (passivation). Moreover, it is important to note that, according to the present invention, the water is nebulized and evaporated in several steps, such that, under the abovementioned conditions of temperature, flow rate of air and flow rate of water, the maximum quantity of nebulized water in contact with the granulate is not great and does not exceed, in each step, 0.5% by weight of the mass being processed.

The effervescent granulate thus prepared is then mixed in a rotating-body mixer with lubricants such as, for example, L-leucine, and flavourings, and is then used for preparing tablets.

Typical features of the mixture thus obtained are, before tabletting, as follows:
residual moisture content ≤ 1.0%;
run-out (flow) time of the granulate from a glass funnel (walls inclined at 60° and outlet diameter of 9 mm) < 20 sec.;
tangent of the angle of rest ≤ 1; and
Carr index ≤ 20.

In a second aspect, the present invention relates to an effervescent granulate according to claim 7. Preferably, the said granulate also comprises from 5 to 18% by weight of sorbitol, about 2% by weight of L-leucine and from 5 to 11% by weight of flavourings.

Typically, the ferrous gluconate is in the form of the dihydrate and the citric acid is in anhydrous form.

In a third aspect, the present invention relates to a tablet according to claim 10.

Depending on the content of active principle, the punch used to produce the said tablet will be one with or without reliefs capable of impressing suitable pre-splitting incisions in the form, for example, of a mid-line incision or a T-shaped incision.

Typically, the tablet according to the present invention has a hardness of between 90 and 180 N, a force of expulsion from the die < 400 N (which indicates good lubrication), a disintegration time ≤ 5 minutes, a divalent iron titre equal to 95-105% of the declared value and a trivalent iron content ≤ 1.25% (relative to the content of ferrous gluconate).

The examples which follow are intended to illustrate the present invention without, however, limiting it in any way.

### EXAMPLE 1

### Effervescent tablet comprising 37.5 mg of iron

### a) Preparation of the effervescent granulate

A mixture of ferrous gluconate dihydrate (32.50 kg), anhydrous citric acid (119.00 kg), sodium bicarbonate (70.00 kg), sorbitol (50.25 kg) and sodium saccharin (2.25 kg) was screened using a vibrating pneumatic screen with a mesh aperture size of 3 mm.

The screened mixture was placed in a fluid-bed granulator (1200 litre capacity) and maintained in suspension by means of a stream of dried air (residual moisture content < 1.0 g/kg; temperature = 70°C; throughput = 3000 m³/hour).

Once the said mixture reached a temperature of 35°C, granulation was started by means of nebulizing purified water. The said nebulizing was stopped when the temperature fell to about 32°C and restarted when the temperature approached 38°C. These steps were repeated until the granulating water (6 kg) was used up. The nebulizing was carried out by passing the water (throughput of 1 litre/min.) through nebulizer nozzles (1.0 mm in diameter), at a pressure of 3 atmospheres.

The granulate was then dried by means of a flow of air heated to 80°C, until the residual moisture content, measured by the Karl Fischer method, of the granulate was ≤ 0.5%. The granules thus obtained were then cooled and removed from the granulator in an environment with a residual moisture content ≤ 20 g/kg and at a temperature of 21-22°C.

The granulate thus obtained had the following features:

| | |
|---|---|
| Residual moisture content | 0.30% |
| Average particle size | 213 µ |
| Flow time^{(*)} | 17 sec. |

| | |
|---|---|
| ^{(*)} The flow time was measured as the run-out time of the granulate (100 g) from a glass funnel with walls inclined at 60° and with an outlet diameter of 9 mm. | |

| | |
|---|---|
| Tangent of the angle of rest | 0.686 |
| Carr index | 13.64 |
| Apparent specific weight | 0.909 g/ml |

### b) Preparation of the mixture before tabletting

L-leucine (6.00 kg), lemon flavouring (10.00 kg) and orange flavouring (10.00 kg) were added to the granulate (274.00 kg) obtained as described in point a) above. This mixture was kept stirring in a rotating-body mixer until a uniform mixture was obtained.

### c) Tabletting

The abovementioned mixture was tabletted using steel punches (force = 50 kN) to form tablets of 3.0 g each.

The tablets thus obtained had the following features:

| | |
|---|---|
| Colour | green-grey |
| Shape | circular, flat |
| Iron content | 37.5 mg |
| Residual moisture content | 0.324% |
| Hardness | 152.7 N |
| Disintegration time | 80 seconds |
| Force of extraction from the die | 200 N |
| Divalent iron titre | 100.6% |
| Trivalent iron titre | 0.83% |

The said tablets were packaged in rooms with a residual moisture content ≤ 20 g/kg and at a temperature of 21-22°C.

### EXAMPLE 2

### Effervescent tablet containing 40.0 mg of iron

### a) Preparation of the effervescent granulate

The process was performed in a manner similar to that described in Example 1 above, except that the mixture placed in the fluid-bed granulator had the following composition: ferrous gluconate dihydrate (34.75 kg), anhydrous citric acid (119.00 kg), sodium bicarbonate (70.00 kg), sorbitol (48.00 kg) and sodium saccharin (2.25 kg).

The granulate thus obtained had the following features:

| | |
|---|---|
| Residual moisture content | 0.34% |
| Average particle size | 278 µ |
| Flow time | 10 sec. |
| Tangent of the angle of rest | 0.500 |
| Carr index | 19.23 |
| Apparent specific weight | 0.769 g/ml |

### b) Preparation of the mixture before tabletting

The process was performed as in Example 1 above.

### c) Tabletting

The process was performed as in Example 1 above.

The tablets thus obtained had the following features:

| | |
|---|---|
| Colour | green-grey |
| Shape | circular, flat |
| iron content | 40.0 mg |
| Residual moisture content | 0.270% |
| Hardness | 128 N |
| Disintegration time | 85 seconds |
| Force of extraction from the die | 230 N |
| Divalent iron titre | 99.9% |
| Trivalent iron titre | 0.10% |

### EXAMPLE 3

### Effervescent tablet containing 75.0 mg of iron

### a) Preparation of the effervescent granulate

The process was performed in a manner similar to that described in Example 1 above, except that the mixture placed in the fluid-bed granulator had the following composition: ferrous gluconate dihydrate (65.00 kg), anhydrous citric acid (98.50 kg), sodium bicarbonate (58.50 kg), sorbitol (38.00 kg) and sodium saccharin (4.00 kg).

The granulate thus obtained had the following features:

| | |
|---|---|
| Residual moisture content | 0.40% |
| Average particle size | 331 µ |
| Flow time | 9 sec. |
| Tangent of the angle of rest | 0.552 |
| Carr index | 14.62 |
| Apparent specific weight | 0.769 g/ml |

### b) Preparation of the mixture before tabletting

L-leucine (6.00 kg), lemon flavouring (15.00 kg) and orange flavouring (15.00 kg) were added to the granulate (264.00 kg) obtained as described in the abovementioned point a).

### c) Tabletting

The process was performed in a manner similar to that in Example 1 above, except that the abovementioned mixture was tabletted using a steel punch capable of impressing a mid-line pre-splitting incision on one face.

The tablets thus obtained had the following features:

| | |
|---|---|
| Colour | green-grey |
| Shape | circular, flat with mid-line pre-splitting incision |
| Iron content | 75.0 mg |
| Residual moisture content | 0.301% |
| Hardness | 100.2 N |
| Disintegration time | 93 seconds |
| Force of extraction from the die | 210 N |
| Divalent iron titre | 102.9% |
| Trivalent iron titre | 0.55% |

### EXAMPLE 4

### Effervescent tablet containing 80.0 mg of iron

### a) Preparation of the effervescent granulate

The process was performed in a manner similar to that described in Example 1 above, except that the mixture placed in the fluid-bed granulator had the following composition: ferrous gluconate dihydrate (69.50 kg), anhydrous citric acid (98.50 kg), sodium bicarbonate (58.50 kg), sorbitol (33.50 kg) and sodium saccharin (4.00 kg).

The granulate thus obtained had the following features:

| | |
|---|---|
| Residual moisture content | 0.18% |
| Average particle size | 286 µ |
| Flow time | 6 sec. |
| Tangent of the angle of rest | 0.550 |
| Carr index | 15.38 |
| Apparent specific weight | 0.769 g/ml |

### b) Preparation of the mixture before tabletting

L-leucine (6.00 kg), lemon flavouring (15.00 kg) and orange flavouring (15.00 kg) were added to the granulate (264.0 kg) obtained as described in the abovementioned point a).

### c) Tabletting

The process was performed in as in Example 3 above.

The tablets thus obtained had the following features:

| | |
|---|---|
| Colour | green-grey |
| Shape | circular, flat with mid-line pre-splitting incision |
| Iron content | 80.0 mg |
| Residual moisture content | 0.459% |
| Hardness | 108 N |
| Disintegration time | 90 seconds |
| Force of extraction from the die | 200 N |
| Divalent iron titre | 100.6% |
| Trivalent iron titre | 0.48% |

### EXAMPLE 5

### Effervescent tablet containing 112.5 mg of iron

### a) Preparation of the effervescent granulate

The process was performed in a manner similar to that described in Example 1 above, except that the mixture placed in the fluid-bed granulator had the following composition: ferrous gluconate dihydrate (97.50 kg), anhydrous citric acid (89.00 kg), sodium bicarbonate (53.50 kg), sorbitol (20.00 kg) and sodium saccharin (4.00 kg).

The granulate thus obtained had the following features:

| | |
|---|---|
| Residual moisture content | 0.34% |
| Average particle size | 280 µ |
| Flow time | 6 sec. |
| Tangent of the angle of rest | 0.552 |
| Carr index | 15.33 |
| Apparent specific weight | 0.909 g/ml |

### b) Preparation of the mixture before tabletting

L-leucine (6.00 kg), lemon flavouring (15.00 kg) and orange flavouring (15.00 kg) were added to the granulate (264.00 kg) obtained as described in the abovementioned point a).

### c) Tabletting

The process was performed as in Example 3 above, except that the abovementioned mixture was tabletted using a steel punch capable of impressing a T-shaped pre-splitting incision on one face.

The tablets thus obtained had the following features:

| | |
|---|---|
| Colour | green-grey |
| Shape | circular, flat with T-shaped pre-splitting incision |
| Iron content | 112.5 mg |
| Residual moisture content | 0.290% |
| Hardness | 107 N |
| Disintegration time | 120 seconds |
| Force of extraction from the die | 280 N |
| Divalent iron titre | 101.0% |
| Trivalent iron titre | 0.87% |

## Claims

1. A method for preparing an effervescent granulate comprising a pharmacologically active ingredient, and citric acid and sodium bicarbonate as effervescent system, by means of wet granulation in a fluid-bed granulator, comprising the steps of:
I. feeding a mixture comprising said active ingredient, citric acid and sodium bicarbonate into the said granulator which is simultaneously fed with a flow of air heated to about 70°C and having a moisture content of < 1.0 g/kg;
II. wetting the said mixture with nebulized water;
III. evaporating off the nebulizing water;
**characterized in that** said mixture includes ferrous gluconate as said pharmacologically active ingredient, and said step II is carried out when said mixture has reached a temperature of about 35°C, and in said step III the temperature of said mixture is kept substantially constant at 35°C, the method further comprising the following steps:
IV. repeating steps II and III until a granulate with the desired features is obtained; and
V. drying the said granulate with air heated to about 80°C until its moisture content is ≤ 0.5% by weight.

2. A method according to claim 1, **characterized in that**, in step I, the magnitude of the air flow rate is between 3200 and 3800 m³/hour for each batch of about 265 kg of mixture to be granulated.

3. A method according to claim 1 or 2, **characterized in that**, in step II, the flow rate of nebulized water is preferably between 500 and 2000 ml/min.

4. A method according to any one of the preceding claims 1 to 3, **characterized in that**, in step II, the pressure of the flow of nebulized water is about 3 atmospheres (3 x 1.013 bar).

5. A method according to any of claims 1 to 4, **characterized in that** the total amount of water used in step II does not exceed 2% by weight of the mixture to be granulated.

6. A method according to any of claims 1 to 5, **characterized in that** the amount of nebulized water in each step does not exceed 0.5% by weight of the mass being processed.

7. An effervescent granulate obtained according to any of the preceding claims, comprising from 7 to 35% by weight of ferrous gluconate, from 25 to 45% by weight of citric acid and from 15 to 25% by weight of sodium bicarbonate.

8. An effervescent granulate according to claim 7, **characterized in that** it also comprises from 5 to 18% by weight of sorbitol, about 2% by weight of L-leucine and from 5 to 11% by weight of flavourings.

9. A granulate according to claim 7, **characterized in that** the ferrous gluconate is in the form of the dihydrate.

10. A tablet obtained by tabletting an effervescent granulate which is obtained according to any one of the preceding claims 1 to 6, the said tablet comprising from 7 to 35% by weight of ferrous gluconate, from 25 to 45% by weight of citric acid, from 15 to 25% by weight of sodium bicarbonate, from 5 to 18% by weight of sorbitol, about 2% by weight of L-leucine and from 5 to 11% by weight of flavourings.

## Patentansprüche

1. Verfahren zur Herstellung eines Brausegranulats, das einen pharmakologisch aktiven Bestandteil, Zitronensäure und Natriumbicarbonat als Brausesystem umfasst, mittels Nassgranulation in einem Fliessbettgranulator, das die folgenden Schritte umfasst:
(i) Zuführen einer Mischung, die den aktiven Bestandteil, Zitronensäure und Natriumbicarbonat umfasst, in den Granulator, dem gleichzeitig ein auf ca. 70°C erwärmter und einen Feuchtigkeitsgehalt von < 1,0 g/kg aufweisender Luftfluss zugeführt wird;
(ii) Benetzen der Mischung mit vernebeltem Wasser;
(iii) Verdampfen des vernebelten Wassers;
**dadurch gekennzeichnet, dass** die Mischung Eisen(II)gluconat als pharmakologisch aktiven Bestandteil einschliesst und Schritt (ii) durchgeführt wird, wenn die Mischung eine Temperatur von etwa 35°C erreicht hat, und in Schritt (iii) die Temperatur der Mischung im wesentlichen konstant bei 35°C gehalten wird, und das Verfahren umfasst ferner die folgenden Schritte:
(iv) Wiederholen der Schritte (ii) und (iii) bis ein Granulat mit den gewünschten Merkmalen erhalten wird; und
(v) Trocknen des Granulats mit auf etwa 80°C erwärmter Luft, bis der Feuchtigkeitsgehalt ≤ 0,5 Gew.% ist.

2. Verfahren gemäss Anspruch 1, **dadurch gekennzeichnet, dass** in Schritt (i) die Luftflussmenge zwischen 3.200 und 3.800 m³/std für jeden Ansatz von etwa 265 kg zu granulierender Mischung liegt.

3. Verfahren gemäss Anspruch 1 oder 2, **dadurch gekennzeichnet, dass** in Schritt (ii) die Flussgeschwindigkeit des vernebelten Wassers vorzugsweise zwischen 500 und 2.000 ml/min beträgt.

4. Verfahren gemäss mindestens einem der vorhergehenden Ansprüche 1 bis 3, **dadurch gekennzeichnet, dass** in Schritt (ii) der Druck des Flusses des vernebelten Wassers etwa 3 Atmosphären (3 x 1,013 bar) beträgt.

5. Verfahren gemäss mindestens einem der Ansprüche 1 bis 4, **dadurch gekennzeichnet, dass** die gesamte in Schritt (ii) verwendete Wassermenge 2 Gew.% der zu granulierenden Mischung nicht übersteigt.

6. Verfahren gemäss mindestens einem der Ansprüche 1 bis 5, **dadurch gekennzeichnet, dass** die Menge an vernebeltem Wasser in jedem Schritt 0,5 Gew.% der zu verarbeitenden Masse nicht übersteigt.

7. Brausegranulat, das erhalten wird nach mindestens einem der vorhergehenden Ansprüche und 7-35 Gew.% Eisen(II)gluconat, 25-45 Gew.% Zitronensäure und 15-25 Gew.% Natriumbicarbonat umfasst.

8. Brausegranulat gemäss Anspruch 7, **dadurch gekennzeichnet, dass** es ferner 5-18 Gew.% Sorbit, etwa 2 Gew.% L-Leucin und 5-11 Gew.% Aromastoffe umfasst.

9. Granulat gemäss Anspruch 7, **dadurch gekennzeichnet, dass** das Eisen(II)gluconat in Form seines Dihydrats vorliegt.

10. Tablette, die erhalten wird durch Tablettieren eines Brausegranulats, das nach mindestens einem der vorhergehenden Ansprüche 1 bis 6 erhalten wird, die Tablette umfasst 7-35 Gew.% Eisen(II)gluconat, 25-45 Gew.% Zitronensäure, 15-25 Gew.% Natriumbicarbonat, 5-18 Gew.% Sorbit, etwa 2 Gew.% L-Leucin und 5-11 Gew.% Aromastoffe.

## Revendications

1. Procédé pour la préparation d'un granulat effervescent comprenant un principe pharmacologiquement actif, et de l'acide citrique et du bicarbonate de sodium, en tant que système effervescent, par le biais d'une granulation humide dans un granulateur en lit fluidisé, comprenant les étapes consistant à :
I. alimenter d'un mélange comprenant ledit principe actif, de l'acide citrique et du bicarbonate de sodium ledit granulateur qui est balayé simultanément avec un flux d'air chauffé à environs 70°C et ayant une teneur en humidité de <1,0 g/kg ;
II. humidifier ledit mélange avec de l'eau nébulisée ;
III. évaporer l'eau nébulisée ;
**caractérisé en ce que** ledit mélange inclut du gluconate ferreux comme étant ledit principe pharmacologiquement actif, et que ladite étape II est effectuée quand ledit mélange atteint une température d'environ 35°C, et dans ladite étape III la température du dit mélange est maintenue sensiblement constante à 35°C, le procédé comprenant de plus les étapes suivantes :
IV. répéter les étapes II et III jusqu'à ce qu'un granulat avec les caractéristiques souhaitées soit obtenu; et
V. sécher ledit granulat avec de l'air chauffé à environ 80°C jusqu'à ce que la teneur en humidité soit ≤ 0,5% en poids.

2. Procédé selon la revendication 1, **caractérisé en ce que**, dans l'étape I, la magnitude du débit d'air est comprise entre 3200 et 3800 m³/heure pour chaque lot d'environ 265 kg de mélange à granuler.

3. Procédé selon la revendication 1 ou 2, **caractérisé en ce que**, dans l'étape II, le débit d'eau nébulisée est compris de préférence entre 500 et 2000 ml/min.

4. Procédé selon l'une quelconque des revendications précédentes 1 à 3, **caractérisé en ce que**, dans l'étape II, la pression du flux d'eau nébulisée est d'environ 3 atmosphères (3x1,013 bar).

5. Procédé selon l'une quelconque des revendications 1 à 4, **caractérisé en ce que** la quantité totale d'eau utilisée dans l'étape II ne dépasse pas 2% en poids du mélange à granuler.

6. Procédé selon l'une quelconque des revendications 1 à 5, **caractérisé en ce que** la quantité d'eau nébulisée dans chaque étape ne dépasse pas 0,5% en poids du substrat traité.

7. Granulat effervescent obtenu selon l'une quelconque des revendications précédentes, comprenant de 7 à 35% en poids de gluconate ferreux, de 25 à 45% en poids d'acide citrique et de 15 à 25% en poids de bicarbonate de sodium.

8. Granulat effervescent selon la revendication 7, **caractérisé en ce qu'**il comprend de 5 à 18% en poids de sorbitol, environ 2% en poids de L-leucine et de 5 à 11% en poids d'aromatisants.

9. Granulat selon la revendication 7, **caractérisé en ce que** le gluconate ferreux est sous sa forme dihydrate.

10. Comprimé obtenu par compression d'un granulat effervescent qui est obtenu selon l'une quelconque des revendications précédentes 1 à 6, ledit comprimé comprenant de 7 à 35% en poids de gluconate ferreux, de 25 à 45% en poids d'acide citrique, de 15 à 25% en poids de bicarbonate de sodium, de 5 à 18% en poids de sorbitol, environ 2% en poids de L-leucine et de 5 à 11% en poids d'aromatisants.
